(19) Europäisches Patentamt
European Patent Office
Office européen des brevets

(11) **EP 1 632 184 B1**

(12) **EUROPEAN PATENT SPECIFICATION**

(45) Date of publication and mention
of the grant of the patent:
**04.04.2012 Bulletin 2012/14**

(51) Int Cl.:
***A61B 8/12*** *(2006.01)*       ***A61B 5/06*** *(2006.01)*
***G06T 3/00*** *(2006.01)*

(21) Application number: **04736120.9**

(22) Date of filing: **04.06.2004**

(86) International application number:
**PCT/JP2004/008151**

(87) International publication number:
**WO 2004/107982 (16.12.2004 Gazette 2004/51)**

(54) **ULTRASONIC ENDOSCOPE**

ULTRASCHALL-ENDOSKOP

ENDOSCOPE A ULTRASONS

(84) Designated Contracting States:
**DE FR GB**

(30) Priority: **06.06.2003 JP 2003162845**

(43) Date of publication of application:
**08.03.2006 Bulletin 2006/10**

(73) Proprietor: **Olympus Corporation
Tokyo 151-0072 (JP)**

(72) Inventor: **KAWASHIMA, Tomonao,
Olympus Corporation
Shibuya-ku, Tokyo 151-0072 (JP)**

(74) Representative: **von Hellfeld, Axel
Wuesthoff & Wuesthoff
Patent- und Rechtsanwälte
Schweigerstrasse 2
81541 München (DE)**

(56) References cited:
**JP-A- 5 324 835**      **JP-A- 10 262 973**
**JP-A- 11 123 187**     **JP-A- 2000 116 655**
**JP-A- 2002 017 729**   **JP-A- 2003 038 492**
**US-A- 5 054 491**      **US-A- 5 671 748**
**US-A1- 2001 035 871**   **US-B1- 6 295 368**

EP 1 632 184 B1

**Description**

Technical Field

The present invention relates to an ultrasonic endoscope device that obtains an optical image and an ultrasonic image of an examinee.

Background Art

Conventionally, various ultrasonic endoscopes are put into the practical use to be inserted from the opening of a patient and scan the body cavity with ultrasonic waves.

The ultrasonic endoscope generally comprises, at the distal end thereof, an ultrasonic vibrator that performs the scanning operation with ultrasonic waves and receives echoes, and an optical observing window that observes an optical image of the surface of the tube cavity of the esophagus, stomach, duodenum, large intestine, and the like.

Generally, an ultrasonic image of the ultrasonic endoscope has a problem that the spatial positional relationship with the optical image is not grasped. Therefore, it is difficult for an operator to understand which portion of the tube cavity, as an ultrasonic image, is displayed on a monitor, and thus needs the skill for precise diagnosis using the ultrasonic image.

The reasons why the spatial positional relationship is not grasped by the ultrasonic endoscope are as follows.

First, a scanned surface with ultrasonic waves is not viewed on the optical image.

Secondly, the scanned surface with ultrasonic waves is not always within the range of field-of-view of the optical image.

Thirdly, in the scanning operation with ultrasonic waves, for the purpose of the reach of the ultrasonic waves to the affected part, degassed water, serving as an ultrasonic medium, is filled in the tube cavity or a balloon is used. Those become an obstacle against the field-of-view of the optical image, and thus the observation with the ultrasonic image is executed after a time, independently of the observation with the optical image.

In order to solve the above-mentioned problems, recently, Japanese Unexamined Patent Application Publication No. 2002-17729 proposes an ultrasonic endoscope device, by which the spatial positional relationship between the optical image and the ultrasonic image is precisely grasped by combining the individually-captured optical image and ultrasonic image and displaying the images.

Further, with the technology proposed in Japanese Unexamined Patent Application Publication No. 2002-17729, the diagnosis of the spread of lesion in the horizontal direction and the diagnosis of the depth of lesion in the vertical direction are simultaneously executed, thereby improving the diagnostic performance of the examination using the ultrasonic endoscope.

The combination of optical image and ultrasonic image needs the correspondence of the positional relationship between both the image data, which is performed in advance. Then, with the technology disclosed in Japanese Unexamined Patent Application Publication No. 2002-17729, as one method, a stereoscopic endoscope measurement system for three-dimensionally grasping the surface of lesion of an examinee based on the optical image and a three-dimensional ultrasonic image restructuring system is combined, the three-dimensional image information of the surface of lesion, obtained by both systems is pattern-matched.

Further, with the technology, the following two methods are proposed to grasp the three-dimensional image information of the surface of lesion based on the optical image.

According to the first method, the stereoscopic measurement is used based on photo cutting with slit-light projection under the basic principle of triangulation. This method is as follows.

First, slit light is generated by using a projecting device with laser beams, as a light source, and is irradiated to a subject surface, thereby projecting the precise shape of the slit light on the subject surface. The distribution of light strength on the subject surface is captured by a scope, thereby obtaining a luminance pattern which changes depending on the shape of subject. The luminance pattern is analyzed, thereby grasping the three-dimensional position having a distance Z to the subject in addition to the X and Y coordinates.

According to the second method, a system for correcting the distortion aberration with a wide-angle lens attached to an endoscope is used.

In the conventional technology for grasping the three-dimensional image information of the surface of lesion based on the optical image, with the stereoscopic measurement based on the photo cutting using the slit-light projection, a specific optical system for guiding laser beams needs to be arranged in the endoscope. Further, with the correction of distortion aberration, the wide-angle lens needs to be attached to the endoscope, and thus a specific endoscope is necessary for stereoscopic three-dimensional endoscope measurement system. Therefore, the endoscope is arranged independently of the endoscope for three-dimensional ultrasonic restructuring system.

Furthermore, with the stereoscopic measurement method, the surface of subject is supposed to be a surface without pattern. In the projection to a surface with an uneven gradation, such as the surface of tube cavity, the three-dimensional position of the surface of subject is not precisely grasped.

In order to combine the optical image and the ultrasonic image by using the ultrasonic endoscope device with the above-mentioned technology and display the combined image, a patient is examined by one system. Thereafter, the endoscope is pulled out and another endoscope is inserted for re-examination. As a result, the examination of the ultrasonic endoscope device takes a long examination time, the troublesomeness of the maintenance of endoscope after/before the examination using cleaning sterilization extremely increases. Thus, a demerit of the burden to the patient is caused. Further, it is not precise in the correspondence of the positional relationship between the optical image and the ultrasonic image.

JP 2000/116655 discloses an endoscope having an optical imaging means and an ultrasonic transducer disposed on an endoscope, each having position sensors disposed thereon. An image obtained by the optical imaging means is displayed side by side on a display with an image obtained by the ultrasonic transducer. The position sensors detect the location and angle of inclination, whereby "UP" directions of both images are oriented in the same direction.

The present invention is devised in consideration of the above circumferences and it is an object of the present invention to provide an ultrasonic endoscope device, in which the positions and directions of the optical image and the ultrasonic image are precisely displayed with a corresponding relationship without replacing the endoscope from the patient.

Disclosure of Invention

In view of the above-mentioned circumstancer, an ultrasonic endoscope according to claim 1 is provided.

An ultrasonic endoscope device according to the present invention includes an optical image obtaining device that obtains an optical image of an examinee, an ultrasonic-image obtaining device that obtains an ultrasonic image of the examinee, a positional-information obtaining device that obtains positional information of the optical image obtaining device to the examinee upon obtaining the optical image, and a matching circuit that matches up an optical image obtained from the optical image obtaining device with the ultrasonic image obtained from the ultrasonic-image obtaining device based on the positional information obtained by the positional-information obtaining device.

Brief Description of the Drawings

Fig. 1 is a block diagram showing the overall configuration of an ultrasonic endoscope device according to a first embodiment of the present invention;

Fig. 2 is an enlarged cross-sectional view showing the distal end of the inserting side of an inserting portion of an endoscope according to the first embodiment of the present invention;

Fig. 3 is a block diagram showing an image processing device according to the first embodiment of the present invention;

Fig. 4 is a block diagram showing a shape matching circuit according to the first embodiment of the present invention;

Fig. 5 is an explanatory diagram of the operation of back-and-forth-by-hand scan according to the first embodiment of the present invention;

Fig. 6 is a conceptual diagram showing three-dimensional image data according to the first embodiment of the present invention;

Fig. 7 is an explanatory diagram of a re-cut-portion of three-dimensional image data according to the first embodiment of the present invention;

Fig. 8 is an explanatory diagram of surface shape data according to the first embodiment of the present invention;

Fig. 9 is an explanatory diagram of the image pickup operation of a target area with an endoscope according to the first embodiment of the present invention;

Fig. 10 is an explanatory diagram of the combination of an optical image and an ultrasonic image according to the first embodiment of the present invention;

Fig. 11 is an explanatory diagram of an image displayed on a monitor according to the first embodiment of the present invention;

Fig. 12 is an explanatory diagram of an ultrasonic endoscope for electronically radial scan and the scanning operation thereof according to the first embodiment of the present invention;

Fig. 13 is an explanatory diagram of an ultrasonic endoscope for convex scan and the scanning operation thereof according to the first embodiment of the present invention;

Fig. 14 is an explanatory diagram of a two-dimensional-array type ultrasonic endoscope and the scanning operation thereof according to the first embodiment of the present invention;

Fig. 15 is a block diagram showing a shape matching circuit according to a second embodiment of the present invention;

Fig. 16 is a block diagram showing a shape matching circuit according to a third embodiment of the present invention;

Fig. 17 is a block diagram showing an image processing device according to a fourth embodiment;

Fig. 18 is a block diagram showing an image processing device according to a fifth embodiment of the present invention; and

Fig. 19 is an explanatory diagram of an image displayed on a monitor according to the fifth embodiment of the present invention.

Best Mode for Carrying Out the Invention

Hereinbelow, a description is given of the embodiments of the present invention with reference to the drawings.

(First embodiment)

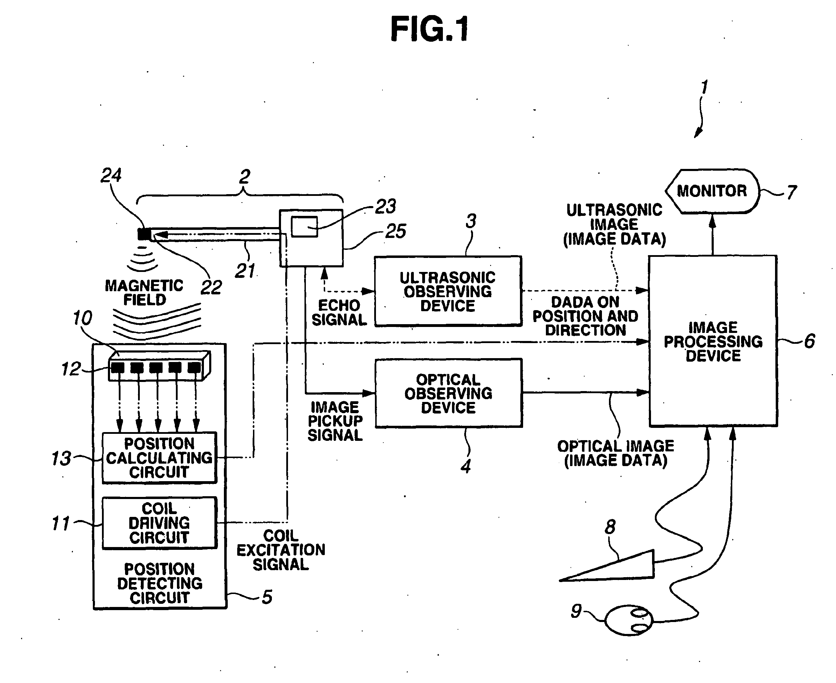

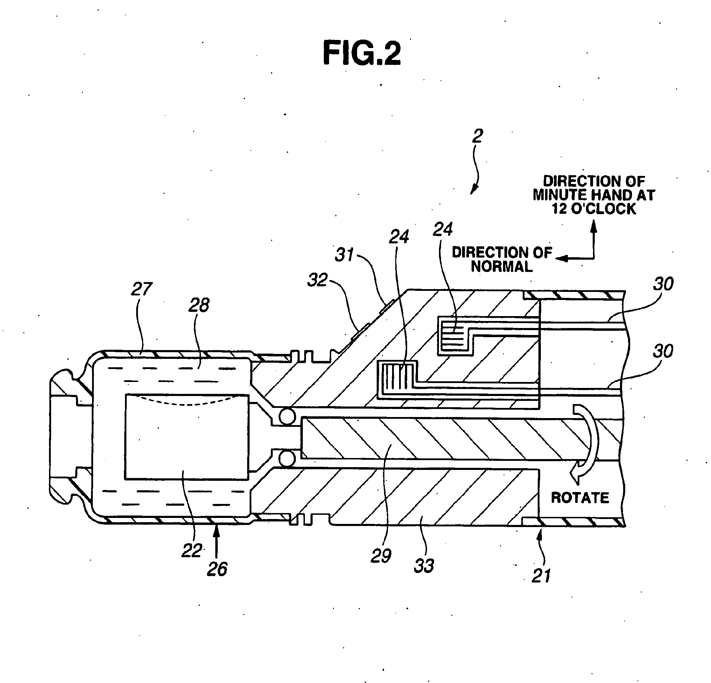

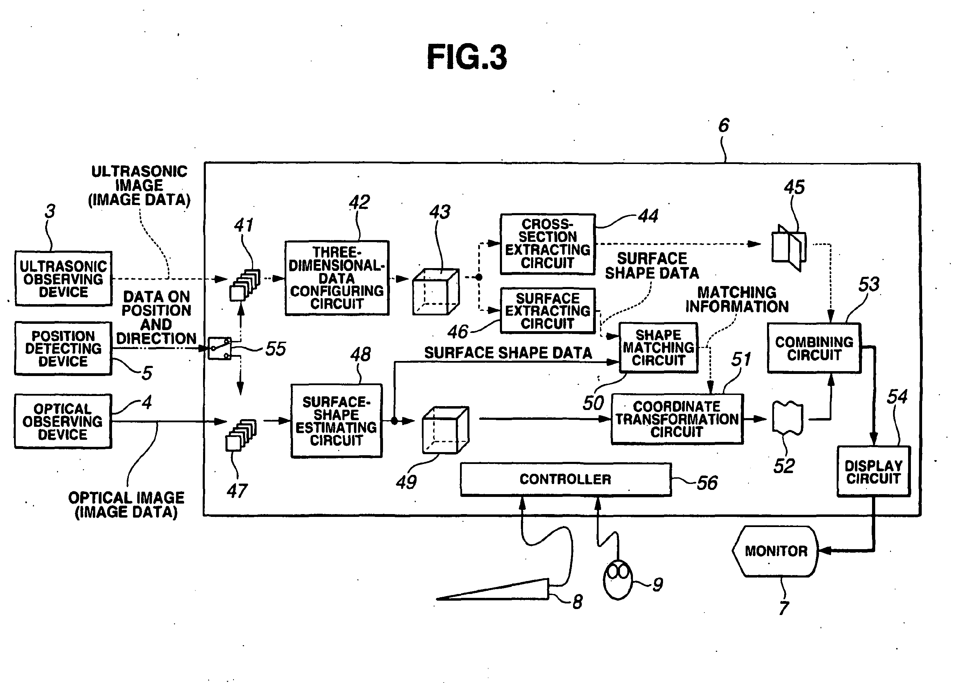

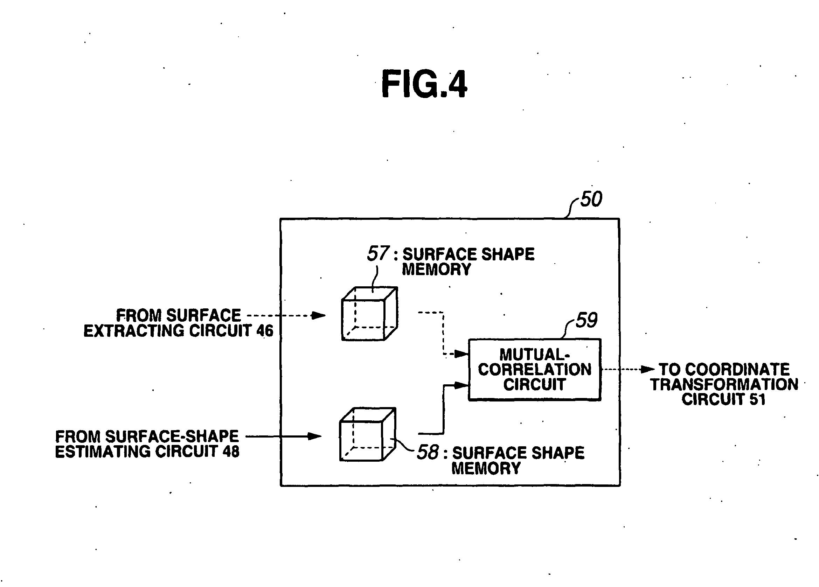

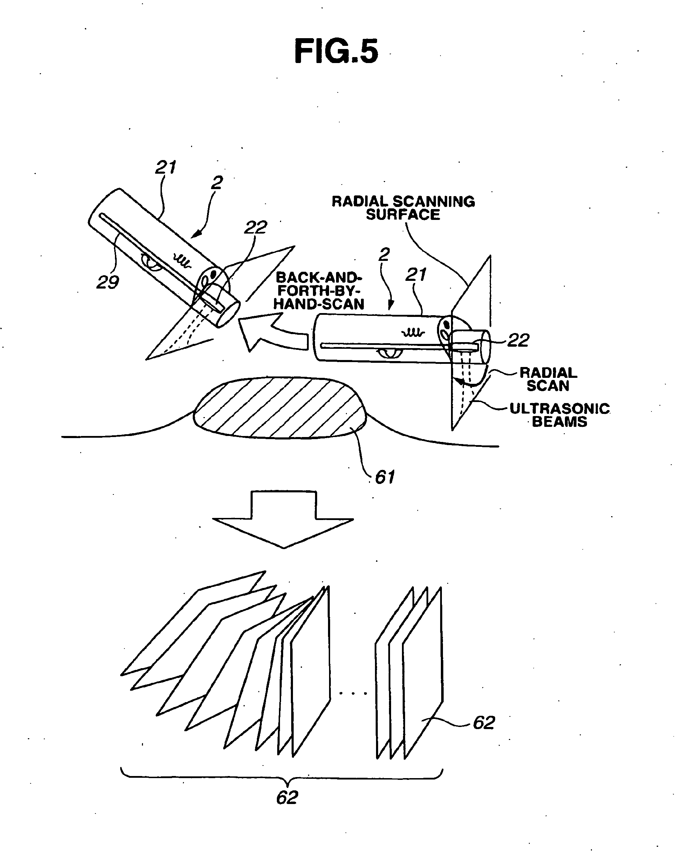

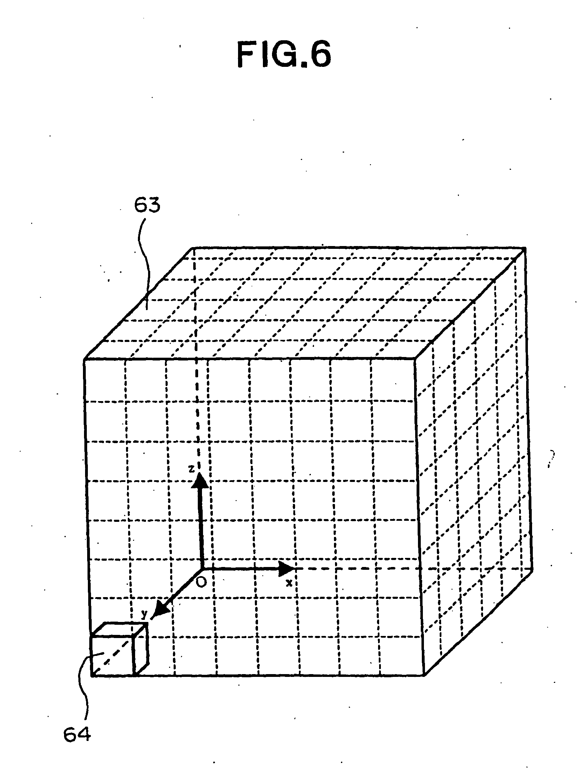

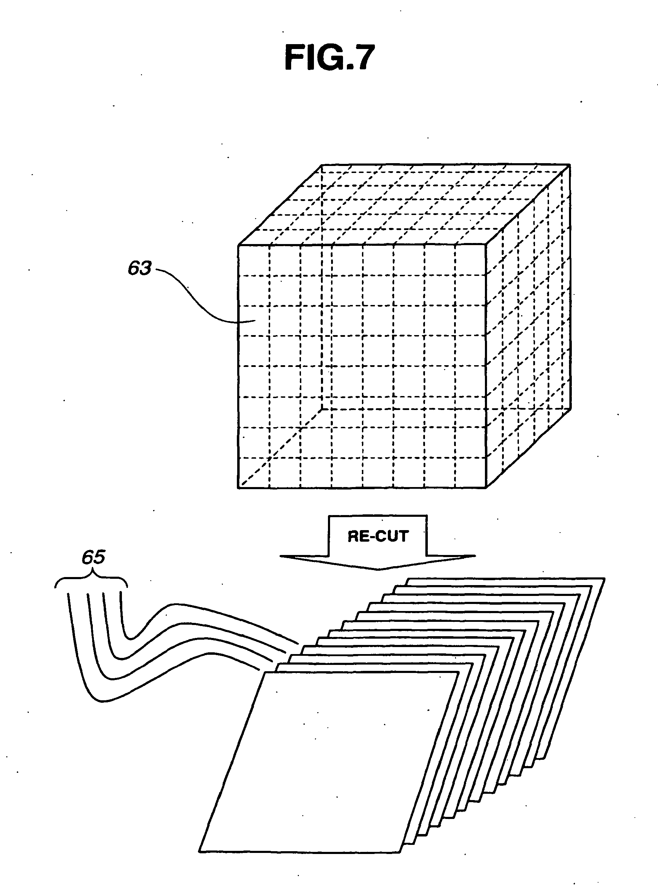

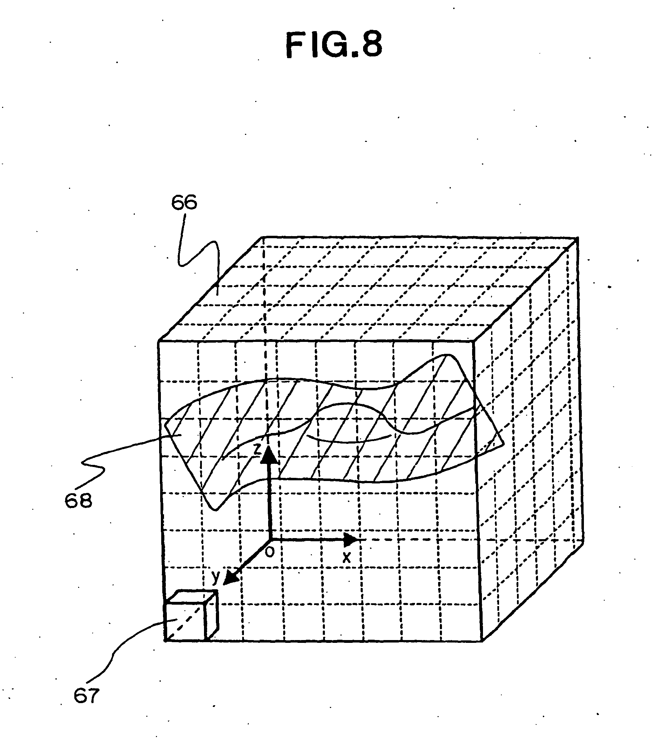

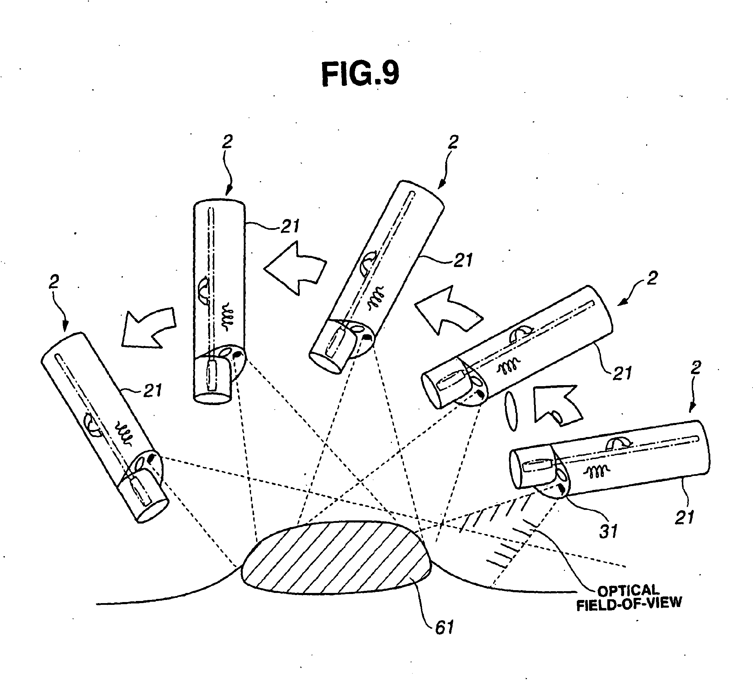

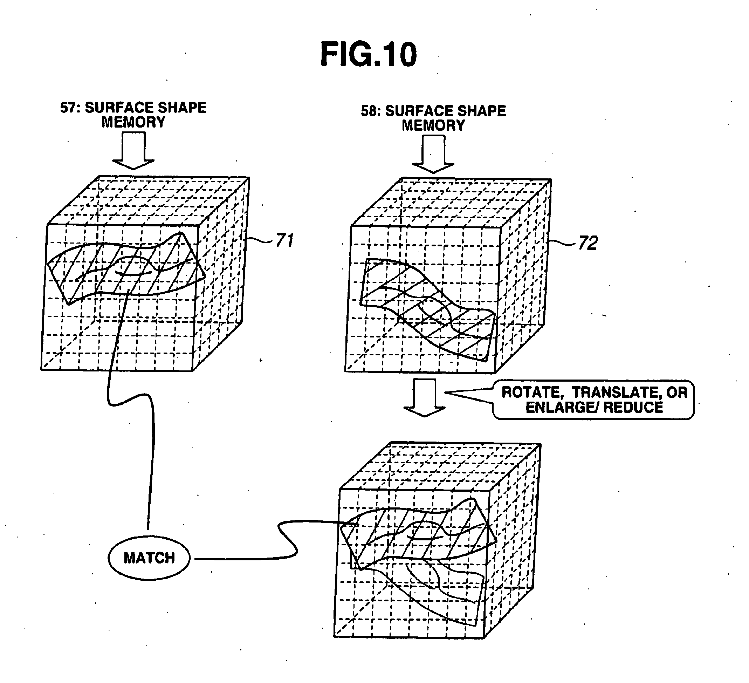

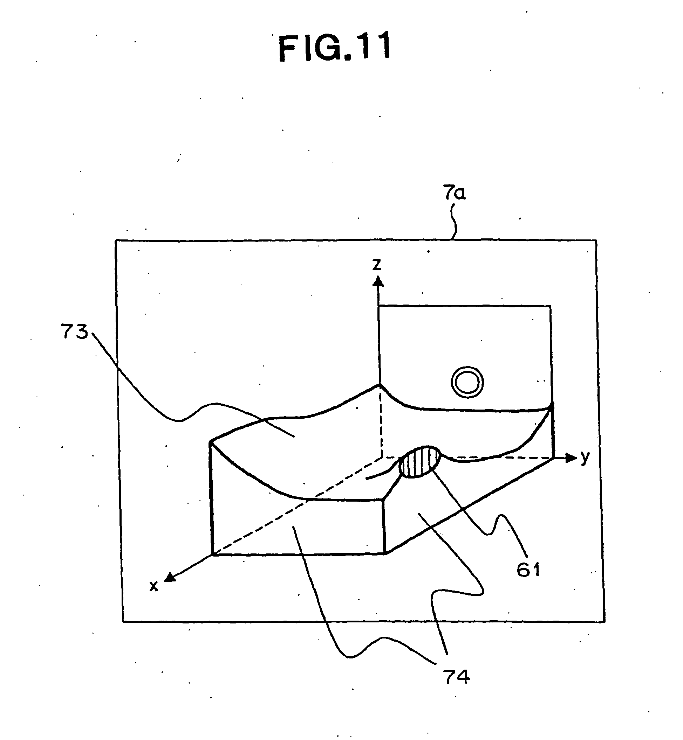

Figs. 1 to 11 relate to a first embodiment of the present invention. Fig. 1 is a block diagram showing the overall configuration of an ultrasonic endoscope device. Fig. 2 is an enlarged cross-sectional view showing the distal end of the inserting side of an inserting portion of an endoscope. Fig. 3 is a block diagram showing an image processing device. Fig. 4 is a block diagram showing a shape matching circuit. Fig. 5 is an explanatory diagram of the operation of back-and-forth-by-hand scan. Fig. 6 is a conceptual diagram showing three-dimensional image data. Fig. 7 is an explanatory diagram of a re-cut-portion of three-dimensional image data. Fig. 8 is an explanatory diagram of surface shape data. Fig. 9 is an explanatory diagram of the image pickup operation of a target area with an endoscope. Fig. 10 is an explanatory diagram of the combination of an optical image and an ultrasonic image. Fig. 11 is an explanatory diagram of an image displayed on a monitor.

(Structure)

Referring to Fig. 1, an ultrasonic endoscope device 1 according to the first embodiment comprises: an ultrasonic endoscope 2; an ultrasonic observing device 3; an optical observing device 4; a position detecting device 5; an image processing device 6; a monitor 7, serving as display means or a display device; a keyboard 8; and a mouse 9.

The ultrasonic endoscope 2 has a continuous arrangement of an inserting portion 21 and an operating portion 25.

The inserting portion 21 comprises a flexible material, and can be inserted in the body cavity of an examinee. The operating portion 25 comprises a motor 23 that drives an ultrasonic vibrator 22 at the distal portion of the inserting portion 21.

The inserting portion 21 comprises, at the distal portion thereof, a sending coil 24 that spatially energizes the magnetic field.

The position detecting device 5, serving as positional-information obtaining means or positional-information obtaining device, comprises: a coil driving circuit 11; a plurality of receiving coils 12; and a position calculating circuit 13.

The coil driving circuit 11 outputs a coil energization signal to the sending coil 24. The plurality of receiving coils 12 are fixed at specific positions by a predetermined arranging method, and sequentially detect the magnetic fields generated by the sending coil 24, thereby outputting an electrical receiving signal.

The position calculating circuit 13 calculates, from the receiving signal outputted from the receiving coils 12, data (hereinafter, referred to as data on the position and direction) indicating the position and the direction of the distal portion of the inserting portion 21.

Incidentally, the plurality of receiving coils 12 is fixed integrally with a rectangular casing. Hereinbelow, the casing and the receiving coils 12 are referred to as a receiving-coil unit 10.

Due to limitations of space, referring to Fig. 1, the receiving coils 12 are fixed in parallel on the straight line in the receiving-coil unit 10. However, actually, the receiving coils 12 are fixed in parallel on the two-dimensional plane or three-dimensional space.

A description will be given of the distal portion of the inserting portion 21 with reference to Fig. 2.

Referring to Fig. 2, a distal portion 26 of the inserting portion 21 comprises an acoustically translucent distal cap 27 containing polymethylpentene or the like. The distal cap 27 includes the ultrasonic vibrator 22, as ultrasonic-image obtaining means or ultrasonic-image obtaining device, and is filled with an ultrasonic transmitting medium 28. The ultrasonic vibrator 22 is connected to a flexible shaft 29 containing a flexible material. The flexible shaft 29 is connected to a rotary shaft of the motor 23 in the operating portion 25 in the endoscope shown in Fig. 1, and is arranged to be rotated in the direction shown by an arrow in Fig. 2. The ultrasonic vibrator 22 outputs an echo signal to the ultrasonic observing device 3 via a signal line (not shown) in the flexible shaft 29, passing through the endoscope operating portion 25.

The inserting portion 21 has, at the distal end thereof, two sending coils 24 constituting solenoid coils for generating the magnetic field in the space. The two sending coils 24 is connected to the coil driving circuit 11 in the position detecting device 5 via a signal line 30. A lead of one of the sending coils 24 is wound like a coil with the axis in the direction described as "direction of minute hand at 12 o'clock", and a lead of the other sending coils 24 is wound like a coil with the axis in the direction described as "the direction of normal".

"The direction of normal" is a direction of the inserting axis of the inserting portion 21, and the "direction of minute hand at 12 o'clock" is perpendicular to the direction of normal. "The direction of normal" matches the direction of normal of the ultrasonic image obtained by radial scan of the ultrasonic vibrator 22. Further, the sending coil 24 wound in the direction of minute hand at 12 o'clock is arranged such that the winding direction of the coil thereof matches the direction of minute hand at 12 o'clock of the ultrasonic image in the direction perpendicular to the direction of normal. Incidentally, the operation of the radial scan of the ultrasonic vibrator 22 will be described later.

In addition, the distal portion 26 of the inserting portion 21 comprises a charge-coupled device solid-state image pickup device camera (hereinafter, referred to as a CCD camera) 31 that captures an optical image with colors; and an image pick-up light irradiating window 32 that irradiates the body cavity with light necessary for image pickup operation by the CCD camera 31.

The CCD camera 31, serving as optical image obtaining means or optical image obtaining device, is connected to the optical observing device 4, and outputs an image pickup signal to the optical observing device 4 via the endoscope operating portion 25, passing through the signal line (not shown) in the inserting portion 21. The optical observing device 4 generates an optical image of the body cavity based on the image pickup signal. Further, image pickup light from a light source device (not shown) reaches the image pick-up light irradiating window 32 via a light guiding channel (not shown), such as an optical fiber, arranged in the inserting portion 21, thereby irradiating the body cavity with image pickup light necessary for image pickup operation of the CCD camera 31.

Further, the inserting portion 21 comprises, at the distal portion thereof, a rigid frame 33 for holding integrally the parts at the distal portion of the inserting portion 21, as shown in Fig. 2.

A detailed description is given of the structure of the image processing device 6 shown in Fig. 1 with reference to Fig. 3.

Referring to Fig. 3, the image processing device 6 comprises: an ultrasonic-image memory 41; a three-dimensional-data configuring circuit 42; a first three-dimensional image memory (hereinafter, simply referred to as three-dimensional image memory 43) with a large capacity; cross-section extracting circuit 44; a cross-section image memory 45; a surface extracting circuit 46, serving as surface shape calculating means or surface shape calculating circuit; an optical-image memory 47; a surface-shape estimating circuit 48, serving as surface shape calculating means or surface shape calculating circuit; a second three-dimensional image memory (hereinafter, simply referred to as three-dimensional image memory 49) with a large capacity; a shape matching circuit 50, serving as matching means; a coordinate transformation circuit 51; a surface image memory 52; a combining circuit 53; a display circuit 54; a switch 55; and a controller 56 for controlling the above units.

The switch 55 switches the output destination of data from the position detecting device 5 to one of the ultrasonic-image memory 41 and the optical-image memory 47. The controller 56 controls the units and circuits in accordance with the input from the keyboard 8 or mouse 9.

Next, a detailed description is given of structure of the shape matching circuit 50 shown in Fig. 3 with reference to Fig. 4.

Referring to Fig. 4, the shape matching circuit 50 comprises a first surface-shape-memory 57; a second surface-shape-memory 58; and a cross-correlation circuit 59.

According to the first embodiment, the position detecting device 5 obtains information on the position and direction of the ultrasonic-image obtaining means with respect to the examinee upon obtaining the ultrasonic image.

(Operation)

Hereinbelow, a description is given of the operation according to the first embodiment.

Referring to Figs. 1, 3 and 4, a solid line indicates the flow of a signal or data of the optical image, and a broken line indicates the flow of a signal or data of the ultrasonic image, a two-dotted-chain line denotes the flow of a signal or data of the position and direction of the inserting portion 21, a thick line denotes the flow of a signal or data of a three-dimensional image, a dotted line indicates the flow of matching information, and a curved arrow denotes the flow of another signal or data.

A description is given of the operation of configuring the ultrasonic image.

The ultrasonic vibrator 22 receives an excitation signal with a pulse voltage from the ultrasonic observing device 3, and converts the received signal to ultrasonic beams, serving as a compression wave of a medium.

The ultrasonic beams are externally radiated to the ultrasonic endoscope 2, via the ultrasonic transmitting medium 28 and the distal cap 27, and a reflecting echo from the examinee is returned to the ultrasonic vibrator 22, passing through a channel reverse to that of the ultrasonic beams.

The ultrasonic vibrator 22 converts the reflecting echo into an electrical echo signal, and transmits the converted signal to the ultrasonic observing device 3, via a channel reverse to that of the excitation signal. Further, the operation repeats and the motor 23 in the operating portion 25 is rotated, thereby rotating the flexible shaft 29 and the ultrasonic vibrator 22 in the direction shown by a block arrow in Fig. 2. Therefore, the ultrasonic beams are sequentially radially radiated in the plane (hereinafter, referred to as a radial-scan plane) perpendicular to the axial direction of the inserting portion 21 of the ultrasonic endoscope 2, and so-called mechanical radial scan is realized.

Hereinbelow, the mechanical radial scan is simply referred to as radial scan.

The ultrasonic observing device 3 performs the publicly known processing including envelope detection, logarithmic amplification, A/D conversion, and scan and conversion (conversion from image data on the polar coordinate system, generated by the radial scan, to the image data on the orthogonal coordinate system) on the echo signal from the ultrasonic vibrator 22, and configures image data of the ultrasonic image (hereinafter, simply referred to as an ultrasonic image). The ultrasonic image is outputted to the ultrasonic-image memory 41 in the image processing device 6.

Next, a description is given of the operation for configuring the optical image.

The CCD camera 31 generates an image pickup signal based on image pickup information on the surface of the body cavity. Specifically, the CCD camera 31 converts the received light into an electrical image pickup signal. Then, the CCD camera 31 outputs the image pickup signal to the optical observing device 4. The optical observing device 4 configures image data of the optical image (hereinafter, simply referred to as an optical image) based on the image pickup signal. The optical image is outputted to the optical-image memory 47 in the image processing device 6.

Next, a description is given of the operation for calculating data on the position and direction of the distal portion of the inserting portion 21.

The coil driving circuit 11 sequentially outputs a coil excitation signal to the sending coils 24. The sending coils 24 generate the magnetic field in the space.

The receiving coils 12 sequentially detect the magnetic field, and output an electrical receiving signal to the position calculating circuit 13.

The position calculating circuit 13 calculates the data on the position and direction based on the receiving signal, and outputs the calculated data to the image processing device 6. The data on the position and direction includes the position and direction of the receiving-coil unit 10 of the sending coil 24. Specifically, the data on the position and direction includes not only the position of the sending coil 24 but also the direction of the inserting axis of the ultrasonic endoscope 2 (direction shown by "the direction of normal" in Fig. 2) and a specific direction in parallel with the ultrasonic image (direction shown by "direction of minute hand at 12 o'clock" in Fig. 2). Here, the direction of the inserting axis of the ultrasonic endoscope 2 corresponds to the direction of normal of the ultrasonic image.

Further, the ultrasonic observing device 3 according to the first embodiment generates the ultrasonic image such that the direction of minute hand at 12 o'clock in Fig. 2 matches the direction of minute hand at 12 o'clock of the ultrasonic image. After all, the data on the position and direction includes the data indicating directions of normal and minute hand at 12 o'clock of the ultrasonic image.

Next, a description is given of the operation of the image processing device 6.

First, a description is given of the flow of signal or data of the ultrasonic image.

An operator allows the controller 56 to switch 55 via the keyboard 8 or mouse 9. Here, the output destination of the data on the position and direction is set to the ultrasonic-image memory 41.

Thereafter, the operator performs the radial scan and simultaneously pulls-out the inserting portion 21 of the ultrasonic endoscope 2 slowly. Referring to Fig. 5, the distal portion of the inserting portion 21 is slowly moved (hereinafter, the scan method is referred to as back-and-forth-by-hand scan). With the back-and-forth-by-hand scan, a plurality of ultrasonic images 62 are continuously obtained. Referring to Fig. 5, most of the ultrasonic images contain an interest area 61 by executing the back-and-forth-by-hand scan such that the distal portion of the inserting portion 21 is always close to the interest area 61.

The ultrasonic observing device 3 sequentially outputs the above-generated ultrasonic images to the ultrasonic-image memory 41. The controller 56 makes the ultrasonic images to have a corresponding relationship with the data on the position and direction at the input timing, and stores the ultrasonic images in the ultrasonic-image memory 41.

For example, the controller 56 stores the data on the position and direction, as a data of the header or footer of the image data of the ultrasonic image. With the advance of recent digital technology, the ultrasonic observing device 3 can create the ultrasonic image in the radial scan without delay. Recently, the position detecting device 5 can calculate the data on the position and direction without the delay with respect to the transmission of magnetic field. Thus, the ultrasonic-image memory 41 actually stores without delay the data on the position and direction at the timing for obtaining the ultrasonic image and its echo signal.

As mentioned above, the ultrasonic-image memory 41 stores a plurality of continuous ultrasonic images by making a corresponding relationship with the data on the position and direction.

The three-dimensional-data configuring circuit 42 reads the continuous ultrasonic images from the ultrasonic-image memory 41, averages the overlapped portion, performs the interpolation of the ultrasonic image, generates the three-dimensional image data of which address is expressed with the three-dimensional orthogonal coordinate, and outputs the generated data to the three-dimensional image memory 43.

The concept of the three-dimensional image data will be described with reference to Fig. 6.

Referring to Fig. 6, three-dimensional image data 63 comprises cells 64 whose addresses are expressed with the three-dimensional orthogonal coordinate, and the cells 64 have, as data, luminance value obtained based on the echo signal.

The cross-section extracting circuit 44 extracts a large number of cells 64 corresponding to a plurality of proper cross sections from the three-dimensional image data 63, and generates image data (hereinafter, referred to as cross-sectional data) on the cross section.

The cross-sectional data is outputted and stored to the cross-section image memory 45. Incidentally, the operator presets the position and direction of the cross section via the keyboard 8 or mouse 9. According to the first embodiment, a plurality of vertical cross sections perpendicular each other are set for the purpose of a brief description.

Referring to Fig. 7, the surface extracting circuit 46 cuts-out again the three-dimensional image data 63 to a parallel cross-sectional image (hereinafter, referred to as parallel slice-image data 65). Then, the cells corresponding to the surface of tube cavity are extracted from parallel slice-image data 65. The method for extracting the surface from the parallel slice-image data 65 is a publicly known method described in Japanese Unexamined Patent Application Publication No. 10-192 by the present applicant. After that, the surface extracting circuit 46 sets, as 1, the cell corresponding to the surface, and further sets, as 0, the cell corresponding to the portion other than the surface, in addition to the three-dimensional image data 63, thereby generating the binalized surface shape data. The surface extracting circuit 46 outputs the data to the surface shape memory 57 in the shape matching circuit 50.

The concept of the surface shape data will be described in detail with reference to Fig. 8. Incidentally, referring to Fig. 8, for the purpose of a brief description, cells 67 of surface shape data 66 are expressed with coarse mesh and, however, the mesh is actually finely cut so that the extracted surface 68 (comprising the cell having the data starting from 1) is smoothly expressed as shown in Fig. 8.

Next, a description is given of the flow of signal or data of the optical image.

First, the operator allows the controller 56 to switch the switch 55 via the keyboard 8 or mouse 9. Here, the output destination of the data on the position and direction is set to the optical-image memory 47. Thereafter, the operator moves the inserting portion 21 of the ultrasonic endoscope 2 while capturing the optical image. Referring to Fig. 9, images of the interest area 61 are captured at various angles.

The optical observing device 4 sequentially outputs the above-generated optical images to the optical-image memory 47.

The controller 56 allows the optical image to have a corresponding relationship with the data on the position and direction at the input timing of the optical image, and stores the optical image to the optical-image memory 47. For example, the controller 56 sets the data on the position and direction, as data of header or footer of the image data of the optical image, and stores the set data. The optical observing device 4 configures the optical image without the delay with respect to the image pickup operation of the CCD camera 31. The position detecting device 5 calculates the data on the position and direction without the delay with respect to the transmission of magnetic field. Therefore, the optical-image memory 47 actually stores the optical images and the data on the position and direction at the image pickup timing of the optical images.

As mentioned above, the optical-image memory 47 stores a plurality of continuous optical images having a corresponding relationship with the data on the position and direction.

The surface-shape estimating circuit 48 reads a plurality of continuous optical images from the optical-image memory 47, and estimates the surface shape. The method for estimating the surface shape is a publicly known method specifically described in Japanese Unexamined Patent Application Publication No. 11-295618 by the present applicant. According to the disclosed processing method, similarly to the present invention, the position detecting device 5 detects the position and direction of the inserting portion 21. Further, the surface shape of the examinee is precisely estimated by using the optical image from the CCD camera 31.

After that, the surface-shape estimating circuit 48 sets, as 1, the cell corresponding to the surface, and further sets, as 0, the cell corresponding to the portion other than the surface, thereby generating the binalized surface shape data to be outputted to the surface shape memory 58 in the shape matching circuit 50. The conceptual diagram of the surface shape data is similar to that with reference to Fig. 8.

Further, the surface-shape estimating circuit 48 maps to the surface shape the color luminance values of the original optical image, thereby generating the three-dimensional image data of the surface of tube cavity in addition to the surface shape data. Then, the surface-shape estimating circuit 48 outputs the data to the three-dimensional image memory 49. The conceptual diagram of the three-dimensional image data is as described with reference to Fig. 6. The three-dimensional image data comprises cells expressed on the three-dimensional orthogonal coordinate. Each cell has, as data, luminance value R (red), G (green), and B (blue) of the surface of tube cavity obtained from the image pickup signal.

Next, a description is given of the operation of the shape matching circuit 50 and the flow of matching information.

The shape matching circuit 50 compares the surface shape data obtained from the ultrasonic image in the surface shape memory 57 and the surface shape data obtained from the optical image in the surface shape memory 58, and calculates the best data of rotation, translation, or enlargement/reduction of the surface shape data obtained from the optical image so that the surface shape data obtained from the optical image matches the surface shape data from the ultrasonic image. This state is shown in Fig. 10.

Specifically, referring to Fig. 10, surface shape data 72 obtained from the optical image by the cross-correlation circuit 59 is subjected to the transformation, such as rotation, translation, or enlargement/reduction, and a cross-correlation value F with respect to the surface shape data 71 obtained from the ultrasonic image is calculated. This processing is repeated while finely changing the degree of conversion, such as rotation, translation, and enlargement/reduction, thereby calculating Euler angle ($\psi$, $\theta$, $\phi$) for rotation, displacement ($\delta x$, $\delta y$, $\delta z$) of translation, and an enlargement/reduction ratio $\alpha$ when the cross-correlation value becomes maximum. Then, the values when the cross-correlation value becomes maximum are outputted to the coordinate transformation circuit 51, as the matching information.

Hereinbelow, an analytical model will be described. The surface shape data 71 obtained from the ultrasonic image is designated by f(x, y, z), and the surface shape data 72 obtained from the optical image is designated by g(x, y, z). Then, functions have the following values.

$$f(x, y, z) = \begin{cases} 1; (x, y, z) = \text{surface} \\ 0; (x, y, z) = \text{another} \end{cases} \quad \cdots (1)$$

$$g(x, y, z) = \begin{cases} 1; (x, y, z) = \text{surface} \\ 0; (x, y, z) = \text{another} \end{cases} \quad \cdots (2)$$

The surface shape data 72 obtained from the optical image is subjected to the rotation, translation, enlargement/ reduction and then a point (x, y, z) on the surface shape data 72 is coordinate-transformed into a point (x', y', z') by the following expression.

$$\begin{pmatrix} x' \\ y' \\ z' \end{pmatrix} = \alpha Tx(\psi) \, Ty(\theta) \, Tz(\phi) \begin{pmatrix} x \\ y \\ z \end{pmatrix} + \begin{pmatrix} \delta x \\ \delta y \\ \delta z \end{pmatrix} \quad \cdots (3)$$

Here, symbols Tx($\psi$), Ty($\theta$), and Tz($\phi$) denote rotation matrixes around the x axis, y axis, z axis.

From Expression (3), symbols x', y', and z' are expressed as functions of (x, y, z), ($\psi'$, $\theta$, $\phi$), ($\delta x$, $\delta y$, $\delta z$), and $\alpha$.

Then, the cross-correlation value F is given as the function of ($\psi$, $\theta$, $\phi$), ($\delta x$, $\delta y$, $\delta z$), and $\alpha$ as the following expression.

$$F(\psi, \theta, \phi, \delta x, \delta y, \delta z\alpha) = \sum_{(x,y,z)} f(x, y, z) \cdot g(x' \, y' \, z') \quad \cdots (4)$$

The obtaining values satisfy the following and become $\psi o$, $\theta o$, $\phi o$, $(\delta X)o$, $(\delta y)o$, $(\delta Z)o$, $\alpha o$ for maximizing the cross-correlation value F. The cross-correlation circuit 59 repeats the calculation of Expressions (3) and (4) so as to obtain the value while finely changing the value of $(\psi, \theta, \phi, (\delta x, \delta y, \delta z)$, and $\alpha$.

$$F(\psi o,\ \theta o,\ \phi o,\ (\delta x)o,\ (\delta y)o,\ (\delta z)o,\ \alpha o)\ = maxF \quad \cdots (5)$$

Finally, the cross-correlation circuit 59 outputs, to the coordinate transformation circuit 51, $\psi o$, $\theta o$, $\phi o$, $(\delta x)o$, $(\delta y)o$, $(\delta z)o$, and $\alpha o$, as the matching information.

The values are coordinate transformation parameters that match the surface shape data obtained from the ultrasonic image to the surface shape data obtained from the optical image.

Next, a description is given of the combination and display of the three-dimensional image.

The coordinate transformation circuit 51 coordinate-transforms the three-dimensional image data 63 obtained from the optical image in the three-dimensional image memory 49.

In this case, the coordinate transformation circuit 51 rotate, translates, or enlarges/reduces the three-dimensional image data 63 obtained from the optical image based on the matching information from the shape matching circuit 50 such that the surface shape data 72 obtained from the optical image best matches up with the surface shape data 71 obtained from the ultrasonic image with the same method of rotating, translating, or enlarging/reducing the surface shape data 72 obtained from the optical image.

Specifically, the coordinate transformation circuit 51 performs the coordinate transformation processing described in Expression (3) of the three-dimensional image data 63 obtained from the optical image in the three-dimensional image memory 49 based on values $\psi o$, $\theta o$, $\phi o$, $(\delta x)o$, $(\delta y)o$, $(\delta z)o$, and $\alpha o$ from the cross-correlation circuit 59. The coordinate-converted three-dimensional image data (hereinafter, referred to as surface image data) is outputted and stored to the surface image memory 52. As a result of the above-mentioned processing, the coordinate system of the cross-sectional data in the cross-section image memory 45 matches the coordinate system of the surface image data in the surface image memory 52.

The combining circuit 53 combines the cross-sectional data and the surface image data, performs processing of shade erasure and the like, combines a surface 73 obtained from the optical image shown in Fig. 11 and a cross section 74 obtained from the ultrasonic image, and configures the three-dimensional image which displays the interest area 61.

The three-dimensional image is converted into a signal, such as a video signal, so that the display circuit 54 outputs the converted signal to a screen 7a of the monitor 7, and is outputted to the monitor 7.

The monitor 7 displays the three-dimensional image.

(Advantages)

According to the first embodiment, the positions and directions of the optical image and the ultrasonic image are displayed with a corresponding relationship therebetween without replacing the endoscope in the patient. Thus, the examination time is reduced and the maintenance of endoscope before/after the examination, such as cleaning and sterilization is simple, and the burden of patient is reduced.

According to the first embodiment, the surface extracting circuit 46 re-cuts the three-dimensional image data into parallel slice-image data 65 and extracts the surface and the surface shape data 66 is thereafter generated. Further, the surface may be extracted without any changes from the ultrasonic images and may be then interpolated, thereby generating the surface shape data 66. With the structure, the surface extracting method described in detail in Japanese Unexamined Patent Application Publication No. 10-192 is used. Further, any publicly known method for extracting the surface may be used.

According to the first embodiment, the surface-shape estimating circuit 48 estimates the surface shape disclosed in Japanese Unexamined Patent Application Publication No. 11-295618. In addition to the method, according to another method, the position and the direction of the inserting portion 21 may be detected and the surface shape of the examinee may be estimated by using the optical images of the same examinee obtained at different times from the CCD camera 31, that is, by stereoscopy with time difference.

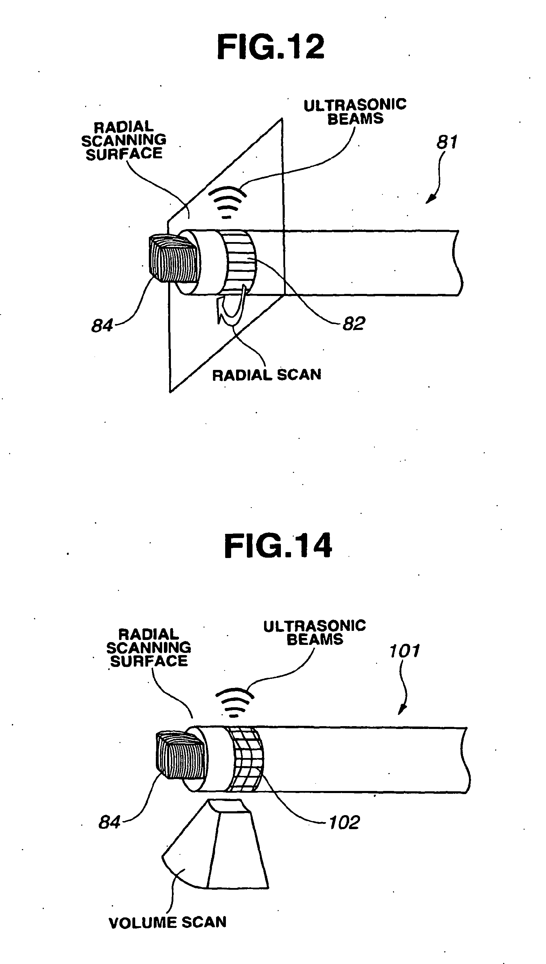

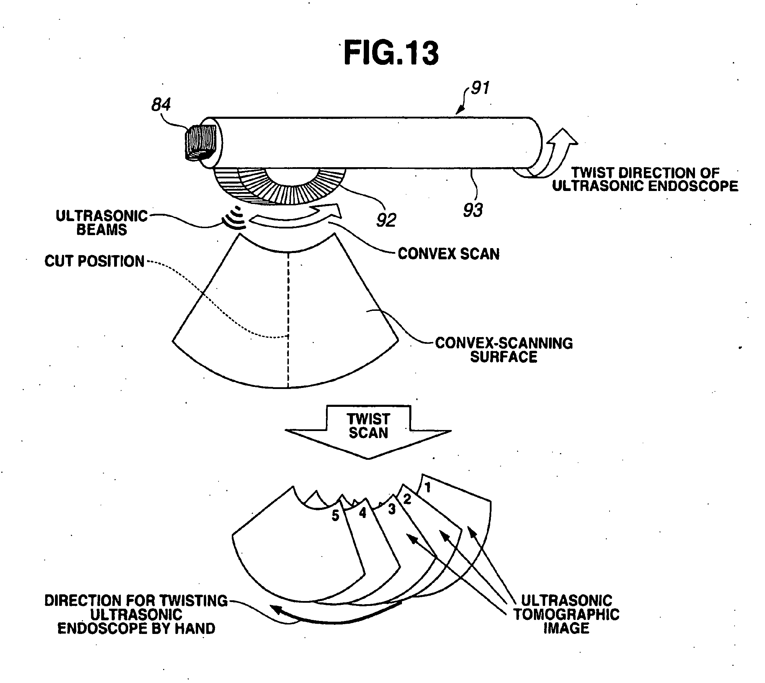

Fig. 12 is an explanatory diagram of an ultrasonic endoscope for electronically radial scan and the scanning operation thereof. Fig. 13 is an explanatory diagram of an ultrasonic endoscope for convex scan and the scanning operation thereof. Fig. 14 is an explanatory diagram of a two-dimensional-array type ultrasonic endoscope and the scanning operation thereof.

According to the first embodiment, as an ultrasonic endoscope, the ultrasonic endoscope 2 for mechanically radial scan is used by mechanically rotating the ultrasonic vibrator 22. However, the present invention is not limited to this, and the ultrasonic endoscope may be an electronically-radial-scan type ultrasonic endoscope 81 having a rectangular ultrasonic vibrator 82 shown in Fig. 12 that is provided like ring or array, or a convex-scan type ultrasonic endoscope 91 shown in Fig. 13 having an ultrasonic vibrator 92 like array along the inserting axis.

With the convex-scan type ultrasonic endoscope 91, in place of the back-and-forth-by-hand scan, the twist scan is used to twist an inserting portion 93 around the inserting portion 93, as the center, shown in Fig. 13.

According to the present invention, referring to Fig. 14, the ultrasonic endoscope may be a two-dimensional-array type ultrasonic endoscope 101 using a two-dimensional-array type ultrasonic vibrator 102 arranged in array with an ultrasonic vibrator on the plane, the ultrasonic vibrator being highly concerned recently.

With the two-dimensional-array type ultrasonic endoscope 101, in place of the back-and-forth-by-hand scan or the twist scan, only the ultrasonic vibrator 102 can perform the scan, not on the two dimension, but on the three dimension (volume scan), thereby obtaining a three-dimensional ultrasonic image at one time. That is, with the above structure, the three-dimensional image data is configured only from the ultrasonic waves, without using the sending coils 24 shown in Fig. 2. Thus, in the scan with ultrasonic waves, the sending coils 24 are not necessary.

According to the first embodiment, as shown in Fig. 2, two sending coils 24 are independently arranged. As shown in Figs. 12, 13, and 14, coils 84 wound to two axes may be integrated. The structure of sending coil can be variously applied. Further, when the sending coils 24 shown in Fig. 1 and the receiving coils 12 are reverse, the data on the position and direction of the distal portion of the inserting portion 21 can be calculated and therefore the problem does not exist. Incidentally, referring to Figs. 12, 13, and 14, although the CCD camera 31 and the image pick-up light irradiating window 32 are not shown, actually, ultrasonic endoscopes 81, 91, and 101 thereof are arranged.

(Second embodiment)

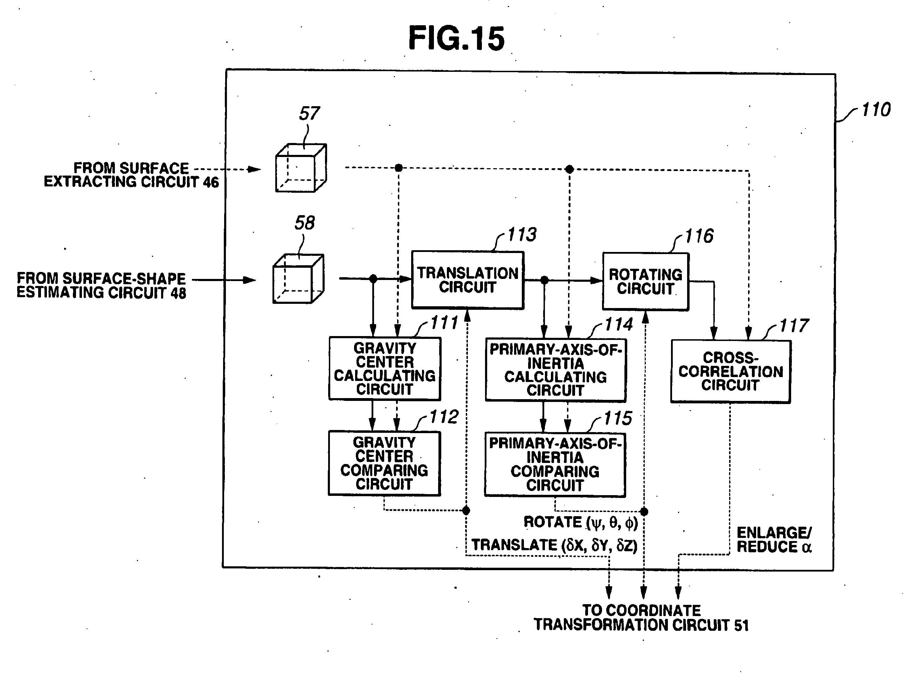

Hereinbelow, a description is given of the structure and the operation of an ultrasonic endoscope device according to the second embodiment with reference to Fig. 15.

Fig. 15 is a block diagram showing a shape matching circuit according to the second embodiment of the present invention.

In the description of the second embodiment with reference to Fig. 15, the same components as those shown in Figs. 1 to 11 according to the first embodiment are designated by the same reference numerals, and a description thereof is omitted.

(Structure)

Referring to Fig. 15, according to the second embodiment, the structure and operation of the shape matching circuit 110 are different from those according to the first embodiment.

The shape matching circuit 110 comprises: a surface shape memory 57; a surface shape memory 58; a gravity center calculating circuit 111 as gravity center calculating means; a gravity center comparing circuit 112; a translation circuit 113; a primary-axis-of-inertia calculating circuit 114, as primary-axis-of-inertia calculating means; a primary-axis-of-inertia comparing circuit 115; a rotating circuit 116; and a cross-correlation circuit 117.

Other structures are the same as those according to the first embodiment.

(Operation)

Referring to Fig. 15, a solid line denotes the flow of signal or data on the optical image, a broken line denotes the flow of signal or data on the ultrasonic image, and a dotted line denotes the flow of matching information.

According to the first embodiment, the cross-correlation circuit performs the rotation, translation, and enlargement/reduction of the surface shape data obtained from the optical image, calculates the cross-correlation value F between the surface shape data obtained from the optical image and the surface shape data obtained from the ultrasonic image, and repeats the above operation by finely changing the degree of rotation, translation, and enlargement/reduction, thereby calculating the Euler angles $(\psi, \theta, \phi)$ for rotation, displacement $(\delta x, \delta y, \delta z)$ of translation, and enlarging/reducing ratio $\alpha$, when the cross-correlation value is maximum.

On the other hand, according to the second embodiment, attention is paid to a fact that the surface shape data obtained from the optical image is the same as the surface shape data obtained from the ultrasonic image, the translation is calculated based on the positional relationship between the gravity centers of both the surface shape data, the rotation is calculated based on the positional relationship between the primary axes of inertia of both the surface shape data, and the enlargement/reduction is calculated by using the cross-correlation circuit 117, thereby calculating the values $(\psi, \theta, \phi)$, $(\delta x, \delta y, \delta z)$, and $\alpha$.

First, the gravity center calculating circuit 111 reads the surface shape data stored in both the surface shape memories, and calculates positional vectors of the gravity centers thereof. The calculation of a positional vector G of the gravity center is given by the following expression.

$$\dot{G} = \sum i \ \dot{I}i \cdot \dot{r}i \qquad \cdots (6)$$

Here, reference symbol i denotes a number of cell forming the surface shape memory, reference symbol ri denotes the positional vector of cell, and reference symbol Ii denotes data of cell (1 to the surface, and 0 to other portions).

The gravity center comparing circuit 112 calculates a difference vector between the positional vectors G of the gravity centers calculated by using both the surface shape data, thereby calculating the positional deviation between both the surface shape data, that is, the displacement ($\delta$x, $\delta$y, $\delta$z) of translation. Thereafter, the gravity center comparing circuit 112 outputs the value to the coordinate transformation circuit 51 and the translation circuit 113.

The translation circuit 113 performs the translation (parallel transition) on the surface shape data 72 obtained from the optical image in the surface shape memory 58, matches the gravity center of the surface shape data 72 and the surface shape data 71 obtained from the ultrasonic image, and outputs the result to the rotating circuit 116 and the primary-axis-of-inertia calculating circuit 114.

The primary-axis-of-inertia calculating circuit 114 reads the surface shape data from the ultrasonic image stored in the surface shape memory 57, and calculates a unit vector of the primary axis of inertia. The primary-axis-of-inertia calculating circuit 114 also calculates a unit vector of the primary axis of inertia of the surface shape data from the optical image having the matched gravity center by using the translation circuit 113. The primary axis of inertia is a set of orthogonal three axes uniquely existed in any rigid member in the normal classical mechanics.

Then, the primary-axis-of-inertia calculating circuit 114 assumes the surface shape data, as the set of cells having a luminance value Ii and positional vector ri, and further replaces the luminance value with the mass, thereby assuming the surface shape data, as the rigid member. The primary-axis-of-inertia calculating circuit 114 calculates the primary axis of inertia from the surface shape data, similarly to the rigid member.

Here, with respect to the surface shape data from the ultrasonic image and the surface shape data from the optical image, the primary-axis-of-inertia calculating circuit 114 calculates a unit vector of the right system of three orthogonal axes to which the primary axis of inertia perpendicularly intersects. The calculation of the primary axis of inertia is publicly known as classical mechanics and linear algebra.

The primary-axis-of-inertia comparing circuit 115 calculates the relationship between the unit vectors of the primary axes of inertia calculated by using both the surface shape data. The relationship between both the surface image data is expressed as an orthogonal matrix having three rows and three columns, thereby the Euler angles ($\psi$, $\theta$, $\phi$) for rotation are calculated. The values correspond to the rotational deviation between both the surface shape data. Thereafter, the primary-axis-of-inertia comparing circuit 115 outputs the values to the coordinate transformation circuit 51 and the rotating circuit 116.

The rotating circuit 116 performs the rotation of the surface shape data obtained from the optical image outputted from the translation circuit 113, matches the direction of the surface shape data obtained from the optical image to that obtained from the ultrasonic image, and outputs the direction to the cross-correlation circuit 117.

The cross-correlation circuit 117 reads surface shape data 71 obtained from the ultrasonic image from the surface shape memory 57. Then, the cross-correlation circuit 117 enlarges or reduces surface shape data obtained from the optical image outputted from the rotating circuit 116, and obtains the cross correlation between both the surface shape data. Further, the above processing is repeated by changing the enlarging/reducing ratio $\alpha$, obtains the enlarging/reducing ratio $\alpha$ to maximize the cross-correlation value, and outputs the obtained ratio to the coordinate transformation circuit 51.

Other operations are the same as those according to the first embodiment.

(Advantages)

According to the first embodiment, as the matching information, the Euler angles ($\psi$, $\theta$, $\phi$) for rotation, the displacement ($\delta$x, $\delta$y, $\delta$z) of translation, and enlarging/reducing ratio $\alpha$ when the cross-correlation value is maximum are all independent variables. According to the second embodiment, the displacement ($\delta$x, $\delta$y, $\delta$z) of translation is calculated by the gravity center calculating circuit 111 and the gravity center comparing circuit 112, the Euler angles ($\psi$, $\theta$, $\phi$) for rotation are calculated by the primary-axis-of-inertia calculating circuit 114 and the primary-axis-of-inertia comparing circuit 115, and the cross-correlation circuit 117 calculates only the enlarging/reducing ratio $\alpha$. Since the processing is generally slow in the cross correlation, the processing according to the second embodiment is faster than that according to the first embodiment.

Other advantages according to the second embodiment are the same as those according to the first embodiment.

(Third embodiment)

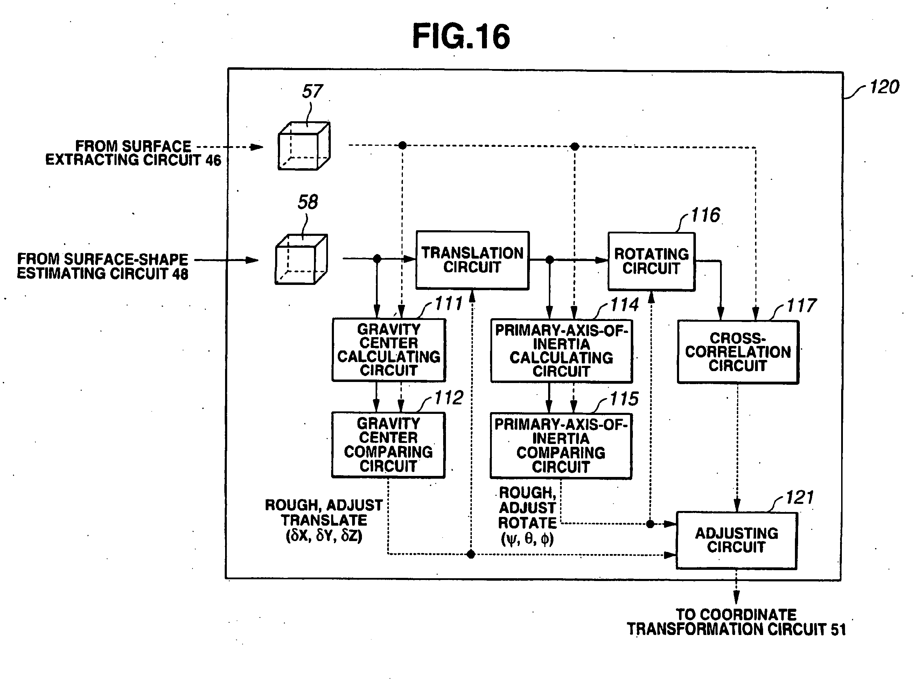

Hereinbelow, a description is given of the structure and the operation of an ultrasonic endoscope device according to a third embodiment with reference to Fig. 16.

Fig. 16 is a block diagram showing a shape matching circuit according to the third embodiment of the present invention.

In the description of the third embodiment with reference to Fig. 16, the same components as those shown in Fig. 15 according to the second embodiment are designated by the same reference numerals, and a description thereof is omitted.

(Structure)

Referring to Fig. 16, according to the third embodiment, the structure and the operation of the shape matching circuit 120 are different from those according to the second embodiment. Only portions different from those according to the second embodiment will be described.

The shape matching circuit 120 additionally has an adjusting circuit 121.

Other structures according to the third embodiment are the same as those according to the second embodiment.

(Operation)

According to the second embodiment, the displacement ($\delta x$, $\delta y$, $\delta z$) of translation, serving as the output of the gravity center comparing circuit 112 and the Euler angles ($\psi$, $\theta$, $\phi$) for rotation, serving as the output of the primary-axis-of-inertia comparing circuit 115 are directly outputted to the coordinate transformation circuit 51. However, according to the third embodiment, the outputs, as coarsely adjusting values, are outputted to the adjusting circuit 121.

Independently of the coarsely adjusting values, the cross-correlation circuit 117 calculates the Euler angles ($\psi$, $\theta$, $\phi$) for rotation, the displacement ($\delta x$, $\delta y$, $\delta z$) of translation, and the enlarging/reducing ratio $\alpha$, similarly to the first embodiment. However, in this case, the Euler angles ($\psi$, $\theta$, $\phi$) for rotation and the displacement ($\delta x$, $\delta y$, $\delta z$) of translation.calculated by the cross-correlation circuit 117 become re-adjusting values obtained by adjustment of the gravity center comparing circuit 112 and the primary-axis-of-inertia comparing circuit 115.

The adjusting circuit 121 calculates precise values of the Euler angles ($\psi$, $\theta$, $\phi$) for rotation and the displacement ($\delta x$, $\delta y$, $\delta z$) of translation from the coarsely adjusting values and fine adjusting values, and outputs the calculated values to the coordinate transformation circuit 51. The adjusting circuit 121 outputs the enlarging/reducing ratio $\alpha$ without change from the cross-correlation circuit 117.

(Advantages)

According to the second embodiment, the coarsely adjusting values, as the matching information, are outputted to the coordinate transformation circuit 51. However, the image pickup range of the body cavity is sometimes finely different between the surface shape data obtained from the optical image and the surface shape data obtained from the ultrasonic image, and the coarsely adjusting values do not sometimes precisely express the Euler angles for rotation and the displacement of translation

According to the third embodiment, the cross-correlation circuit 117 calculates the fine adjusting values. Thus, the shape matching circuit 120 more precisely outputs the matching information, as compared with the case according to the second embodiment. Further, the cross-correlation circuit 117 performs the coarse adjustment before calculating the fine adjusting values, thereby performing the cross-correlation processing while changings the independent variables having limiting changing ranges. Thus, the processing is faster according to the third embodiment, as compared with that according to the first embodiment.

Other structures are the same as those according to the first embodiment.

(Fourth embodiment)

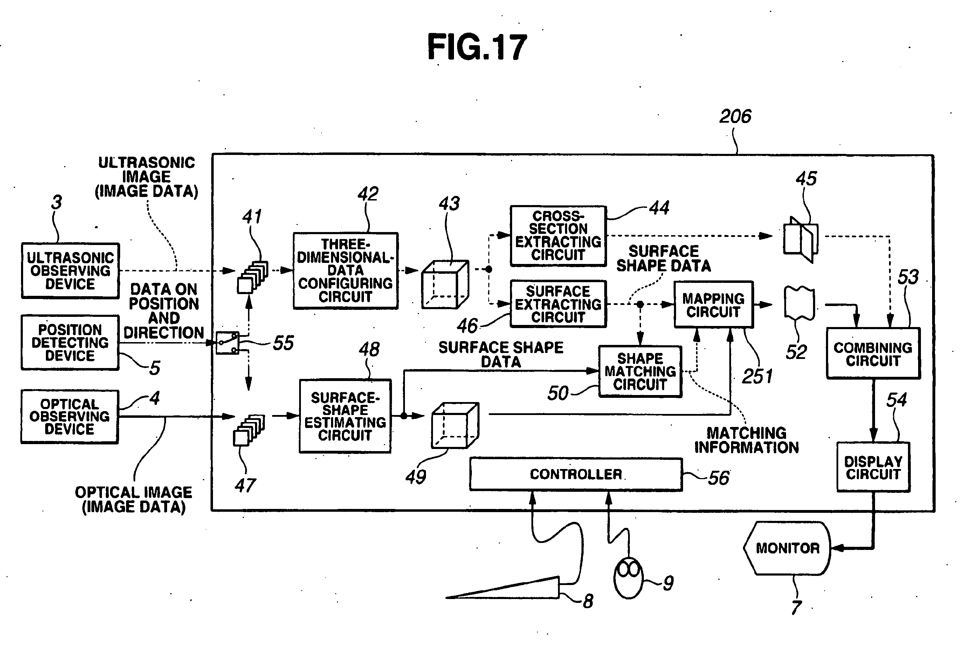

Hereinbelow, a description is given of the structure and the operation of an ultrasonic endoscope device with reference to Fig. 17 according to a fourth embodiment.

Fig. 17 is a block diagram showing an image processing device according to the fourth embodiment.

In the description of the fourth embodiment with reference to Fig. 17, the same components as those shown in Figs. 1 to 11 according to the first embodiment are designated by the same reference numerals, and a description thereof is omitted.

(Structure)

Referring to Fig. 17, according to the fourth embodiment, an image processing device 206 comprises a mapping circuit 251, in place of the coordinate transformation circuit 51 according to the first embodiment.
Other structures are the same as those according to the first embodiment.

(Operation)

According to the first embodiment, the surface of the three-dimensional image shown in Fig. 11 is expressed by using the three-dimensional image data 63 obtained from the optical image without change. However, according to the fourth embodiment, the surface is expressed by mapping the luminance value of the three-dimensional image data obtained from the optical image into the surface shape data obtained from the ultrasonic image. Specifically, it is as follows.
A mapping circuit 251 receives the surface shape data from the surface extracting circuit 46, the matching information from the shape matching circuit 50, the three-dimensional image data 63 from the three-dimensional image memory 49. Of these, the surface shape data is obtained from the ultrasonic image, and the three-dimensional image data has data on the luminance values of R (red), G (green), and B (blue) of the surface of tube cavity obtained from the optical image.
The mapping circuit 251 correlates, based on the matching information, cells of the three-dimensional image data obtained from the optical image to cells of the surface shape data obtained from the ultrasonic image respectively. Then, the luminance value of the three-dimensional image data obtained from the optical image is mapped into the surface shape data obtained from the ultrasonic image, and is outputted to the surface image memory 52.
Other structures are the same as those according to the first embodiment.

(Advantages)

According to the fourth embodiment, the same advantages as those according to the first embodiment are obtained.

(Fifth embodiment)

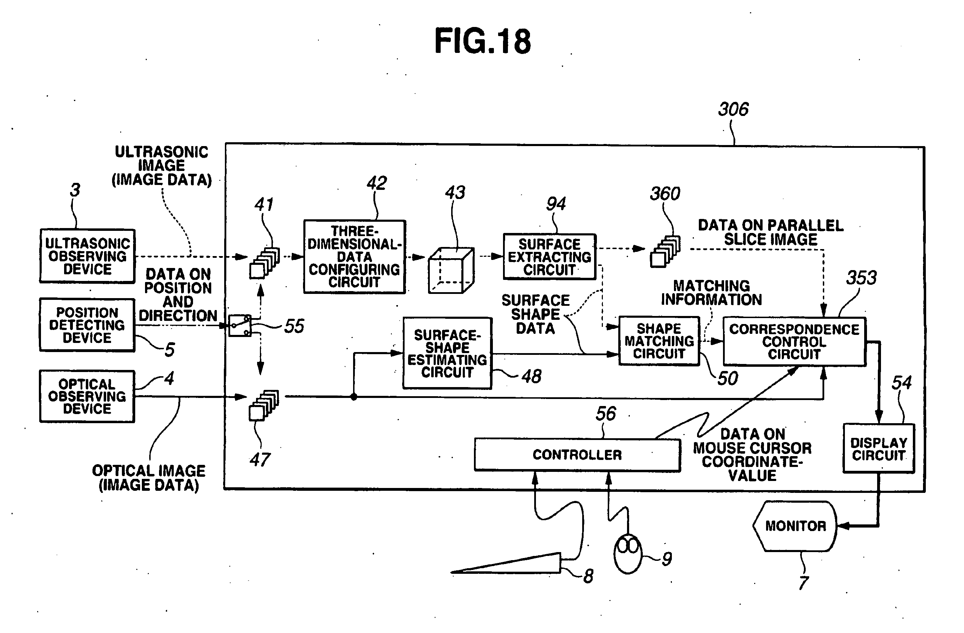

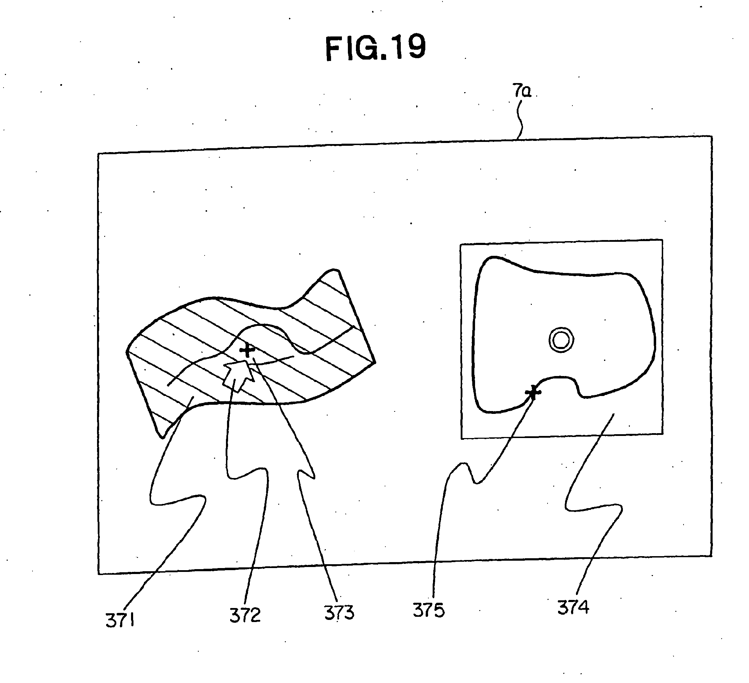

Hereinbelow, a description is given of the structure and the operation of an ultrasonic endoscope device according to a fifth embodiment with reference to Figs. 18 and 19.
Figs. 18 and 19 are related to the fifth embodiment of the present invention, Fig. 18 is a block diagram showing an image processing device and Fig. 19 is an explanatory diagram of an image displayed on a monitor.
In the description of the fifth embodiment with reference to Figs. 18 and 19, the same components as those shown in Figs. 1 to 11 according to the first embodiment are designated by the same reference numerals, and a description thereof is omitted.

(Structure)

Referring to Fig. 18, according to the fifth embodiment, an image processing device 306 comprises a correspondence support circuit 353, in place of the combining circuit 53 according to the first embodiment.
The correspondence support circuit 353 sequentially receives from the controller 56 a coordinate value of the mouse cursor, constituting mouse cursor coordinate-value data, on the screen 7a of the monitor 7 with the mouse 9 being operated by the operator.
According to the fifth embodiment, the image processing device 306 comprises: a parallel-slice image memory 360, in place of the cross-section image memory 45, the three-dimensional image memory 49, the surface image memory 52, and the coordinate transformation circuit 51 according to the first embodiment. The parallel-slice image memory 360 stores all the parallel slice-image data generated by the surface extracting circuit 46.
Other structures are the same as those according to the first embodiment.

(Operation)

Although three-dimensional image shown in Fig. 11 is combined and displayed according to the first embodiment, the original images of the optical image and the ultrasonic image are simultaneously displayed and corresponding points of both the original images are displayed according to the fifth embodiment. This state is shown in Fig. 19.

Specifically, it is as follows.

Referring to Fig. 19, the correspondence support circuit 353 shown in Fig. 18 displays a proper optical image 371 on the left of the screen 7a of the monitor 7. From the optical image 371, the operator selects a desired image by using the keyboard 8 or mouse 9, and moves a mouse cursor 372 on the screen 7a on the monitor 7 by using the mouse 9. Thus, the controller 56 outputs mouse cursor coordinate-value data to the correspondence support circuit 353.

Subsequently, the operator designates one point on the optical image 371 by clicking the mouse. The correspondence support circuit 353 marks a marker 373 on the point.

Subsequently, based on the matching information from the shape matching circuit 50, the correspondence support circuit 353 selects and reads, from a parallel-slice image memory 360, parallel slice-image data including the point corresponding to the marker 373 on the optical image 371. Thereafter, the correspondence support circuit 353 marks a marker 375 on the corresponding point in the parallel slice-image data, and displays a parallel slice image 374 on the right of screen of the monitor 7.

Other operations are the same as those according to the first embodiment.

(Advantages)

According to the first embodiment, the surface generated from the optical image 371 shown in Fig. 11 is combined into the three-dimensional image and the combined image is displayed. In this case, the resolution can be reduced, as compared with that of the original image of the optical image 371. According to the fifth embodiment, an image processing device 306 observes the optical image 371, as the original image with high resolution, and further compares the optical image with the cross section having the luminance value of the ultrasonic image.

(Modification)

According to the fifth embodiment, the data on the parallel slice image is used as ultrasonic data for display. Further, the correspondence support circuit 353 may select, from the ultrasonic-image memory 41, the ultrasonic image of the original image which is closest to the corresponding point to the marker 373 on the optical image 371. With the structure, both the images displayed on the screen of the monitor 7 become the original images. Further, both the images are observed with a corresponding positional relationship between both the original images without deterioration.

As mentioned above, according to the present invention, the positions and the directions of the optical image and the ultrasonic image are displayed with a corresponding relationship without replacing the endoscope from the patient. Therefore, the examining time is reduced, the maintenance of endoscope after/before the examination using the cleaning or sterilization is simple, and the burden to the patient is reduced.

The embodiments of the present invention are described above. However, the present invention is not limited to the embodiments and can be variously changed without departing the spirit of the present invention.

Industrial Applicability

According to the present invention, an ultrasonic endoscope device is provided to make precisely corresponding the position and direction of the optical image and the ultrasonic image with each other and displays the corresponding positions and directions.

**Claims**

1. An ultrasonic endoscope device (1) comprising at a distal end of an ultrasonic endoscope for insertion into a body cavity of an examinee:

   optical image obtaining means (4) that is configured to obtain an optical image of the examinee;
   ultrasonic image obtaining means (3) that is configured to obtain an ultrasonic image of the examinee;
   **characterized by** further comprising:

   positional-information obtaining means (5) that is configured to obtain positional information of the optical image obtaining means (4) with respect to the examinee upon obtaining the optical image; and that obtains the positional information of the ultrasonic-image obtaining means (3) with respect to the examinee upon obtaining the ultrasonic image by using a same device arranged at the distal end of the ultrasonic endoscope; first surface-shape calculating means (48) that is configured to calculate a first surface-shape from the optical image based on (i) a plurality of optical images captured at various angles using the optical image obtaining means; and (ii) the positional information corresponding to each of the plurality of optical images; and

second surface shape calculating means (46) that is configured to calculate a second surface-shape from the ultrasonic image based on the positional information of the ultrasonic image obtaining means; matching means (50) that is configured to perform the matching of the optical, image obtained by the optical image obtaining means (4) and the ultrasonic image obtained by the ultrasonic-image obtaining means (3) by using the first and second surface shapes.

2. The ultrasonic endoscope device according to Claim 1, further comprising: combining means (53, 251) that is configured to combine a three-dimentional image using the surface image data and the cross-sectional data by using a luminance value of the optical image in the surface image data and by using a luminance value of the ultrasonic image in the cross-sectional data.

3. The ultrasonic endoscope device according to Claim 1, further comprising:

   display means (7) that is configured to simultaneously display an image obtained from the optical image and an image obtained from the ultrasonic image; and
   correspondence control means (51) that is configured to calculate a corresponding point on one of the obtained images to an arbitrary point on the other image.

4. The ultrasonic endoscope device according to Claim 1, wherein the matching means is configured to perform the matching by using cross-correlation processing.

5. The ultrasonic endoscope device according to Claim 1, wherein the matching means (50) comprises gravity center calculating means (111) that is configured to calculate the gravity center of the first surface-shape and the second surface-shape.

6. The ultrasonic endoscope device according to Claim 1, wherein the matching means (50) comprises primary-axis-of-inertia calculating means (114) that is configurated to calculate the primary axis of inertia of the first surface-shape and the second surface-shape.

7. The ultrasonic endoscope device according to Claim 1, wherein the matching means (50) comprises gravity center calculating means (111) that is configurated to calculate the gravity center of the first surface-shape and the second surface-shape, or primary-axis-of-inertia calculating means (114) that is configured to calculate the primary axis of inertia of the first surface-shape and the second surface-shape, wherein the gravity center or the primary axis of inertia is calculated before the cross-correlation processing.

8. The ultrasonic endoscope device according to Claim 1, wherein the ultrasonic-image obtaining means (3) is configured to perform volume scanning by itself.

9. The ultrasonic endoscope device according to Claim 8, wherein the ultrasonic-image obtaining means (3) is a two-dimensional-array type ultrasonic vibrator (102) arranged in two-dimensional array with an ultrasonic vibrator (22).

**Patentansprüche**

1. Ultraschall-Endoskopvorrichtung (1), die an einem distalen Ende eines Ultraschall-Endoskops zum Einführen in eine Körperhöhlung einer zu untersuchenden Person umfasst:

   ein optisches Bilderlangungsmittel (4), das zum Erlangen eines optischen Bilds der zu untersuchenden Person konfiguriert ist;
   ein Ultraschallbild-Erlangungsmittel (3), das zum Erlangen eines Ultraschallbilds der zu untersuchenden Person konfiguriert ist;
   **dadurch gekennzeichnet, dass** sie ferner umfasst:

   ein Positionsinformations-Erlangungsmittel (5), das zum Erlangen von Positionsinformation des optischen Bilderlangungsmittels (4) in Bezug auf die zu untersuchende Person nach Erlangen des optischen Bilds konfiguriert ist, und das die Positionsinformation des Ultraschallbild-Erlangungsmittel (3) in Bezug auf die zu untersuchende Person nach Erlangung des Ultraschallbilds unter Verwendung ein und desselben Geräts, das an dem distalen Ende des Ultraschall-Endoskops angeordnet ist, erlangt;

ein erstes Oberflächenform-Berechnungsmittel (48), das zum Berechnen einer ersten Oberflächenform aus dem optischen Bild, basierend auf (i) mehreren aus verschiedenen Winkeln unter Verwendung des optischen Bilderlangungsmittels aufgenommenen optischen Bildern; und (ii) der jedem der mehreren optischen Bilder entsprechenden Positionsinformation, konfiguriert ist; und

ein zweites Oberflächenform-Berechnungsmittel (46), das zum Berechnen einer zweiten Oberflächenform aus dem Ultraschallbild, basierend auf der Positionsinformation des Ultraschallbild-Erlangungsmittels, konfiguriert ist;

ein Abgleichmittel (50), das zum Durchführen des Abgleichs des von dem optischen Bilderlangungsmittel (4) erlangten optischen Bilds und des von dem Ultraschallbild-Erlangungsmittel (3) erlangten Ultraschallbilds unter Anwendung der ersten und zweiten Oberflächenformen konfiguriert ist.

2. Ultraschall-Endoskopvorrichtung nach Anspruch 1, ferner mit:

einem Kombinationsmittel (53, 251), das zum Kombinieren eines dreidimensionalen Bilds mittels der Oberflächen-Bilddaten und der Querschnittsdaten unter Anwendung eines Luminanzwerts des optischen Bildes bei den Oberflächen-Bilddaten und unter Anwendung eines Luminanzwerts des Ultraschallbildes bei den Querschnittsdaten konfiguriert ist.

3. Ultraschall-Endoskopvorrichtung nach Anspruch 1, ferner mit:

einem Anzeigemittel (7), das zum gleichzeitigen Anzeigen eines aus dem optischen Bild erlangten Bildes und eines aus dem Ultraschallbild erlangten Bildes konfiguriert ist; und

einem Korrespondenz-Steuermittel (51), das zum Berechnen eines korrespondierenden Punkts auf einem der erlangten Bilder zu einem beliebigen Punkt auf dem anderen Bild konfiguriert ist.

4. Ultraschall-Endoskopvorrichtung nach Anspruch 1, wobei das Abgleichmittel zur Durchführung des Abgleichs unter Anwendung einer Kreuzkorrelations-Verarbeitung konfiguriert ist.

5. Ultraschall-Endoskopvorrichtung nach Anspruch 1, wobei das Abgleichmittel (50) ein Gravitationszentrum-Berechnungsmittel (111) umfasst, das zum Berechnen des Gravitationszentrums der ersten Oberflächenform und der zweiten Oberflächenform konfiguriert ist.

6. Ultraschall-Endoskopvorrichtung nach Anspruch 1, wobei das Abgleichmittel (50) ein Hauptträgheitsachsen-Berechnungsmittel (114) umfasst, das zum Berechnen der Hauptträgheitsachse der ersten Oberflächenform und der zweiten Oberflächenform konfiguriert ist.

7. Ultraschall-Endoskopvorrichtung nach Anspruch 1, wobei das Abgleichmittel (50) ein Gravitationszentrum-Berechnungsmittel (111) umfasst, das zum Berechnen des Gravitationszentrums der ersten Oberflächenform und der zweiten Oberflächenform konfiguriert ist, oder ein Hauptträgheitsachsen-Berechnungsmittel (114) umfasst, das zum Berechnen der Hauptträgheitsachse der ersten Oberflächenform und der zweiten Oberflächenform konfiguriert ist, wobei das Gravitationszentrum oder die Hauptträgheitsachse vor der Kreuzkorrelations-Verarbeitung berechnet wird.

8. Ultraschall-Endoskopvorrichtung nach Anspruch 1, wobei das Ultraschallbild-Erlangungsmittel (3) zum eigenständigen Durchführen einer Volumenabtastung konfiguriert ist.

9. Ultraschall-Endoskopvorrichtung nach Anspruch 8, wobei das Ultraschallbild-Erlangungsmittel (3) ein Ultraschallvibrator (102) vom Typ mit zweidimensionaler Anordnung ist, der in einer zweidimensionalen Anordnung mit einem Ultraschallvibrator (22) angeordnet ist.

**Revendications**

1. Dispositif d'endoscope à ultrasons (1) comprenant au niveau d'une extrémité distale d'un endoscope à ultrasons pour insertion dans une cavité de corps d'un candidat à l'examen :

un moyen d'obtention d'images optiques (4) qui est configuré pour obtenir une image optique du candidat à l'examen ;

un moyen d'obtention d'images ultrasonores (3) qui est configuré pour obtenir une image ultrasonore du candidat à l'examen ;

**caractérisé en ce qu'**il comprend en outre :

un moyen d'obtention d'information de position (5) qui est configuré pour obtenir une information de position du moyen d'obtention d'images optiques (4) par rapport au candidat à l'examen sur obtention de l'image optique et qui obtient l'information de position du moyen d'obtention d'images ultrasonores (3) par rapport au candidat à l'examen sur obtention de l'image ultrasonore en utilisant un dispositif identique disposé au niveau de l'extrémité distale de l'endoscope à ultrasons ;

un moyen de calcul de première forme de surface (48) qui est configuré pour calculer une première forme de surface à partir de l'image optique sur la base (i) d'une pluralité d'images optiques capturées à divers angles en utilisant le moyen d'obtention d'images optiques ; et (ii) de l'information de position correspondant à chacune de la pluralité d'images optiques ; et

un moyen de calcul de deuxième forme de surface (46) qui est configuré pour calculer une deuxième forme de surface à partir de l'image ultrasonore sur la base de l'information de position du moyen d'obtention d'images ultrasonores ;

un moyen de mise en correspondance (50) qui est configuré pour exécuter la mise en correspondance de l'image optique obtenue par le moyen d'obtention d'images optiques (4) et de l'image ultrasonore obtenue par le moyen d'obtention d'images ultrasonores (3) en utilisant les première et deuxième formes de surface.

2. Dispositif d'endoscope à ultrasons selon la revendication 1, comprenant en outre :

un moyen de combinaison (53, 251) qui est configuré pour combiner une image en trois dimensions en utilisant les données d'image de surface et les données en section transversale en utilisant une valeur de luminance de l'image optique dans les données d'image de surface et en utilisant une valeur de luminance de l'image ultrasonore dans les données en section transversale.

3. Dispositif d'endoscope à ultrasons selon la revendication 1, comprenant en outre :

un moyen d'affichage (7) qui est configuré pour afficher simultanément une image obtenue à partir de l'image optique et une image obtenue à partir de l'image ultrasonore ; et

un moyen de commande de correspondance (51) qui est configuré pour calculer un point de correspondance sur l'une parmi les images obtenues avec un point arbitraire sur l'autre image.

4. Dispositif d'endoscope à ultrasons selon la revendication 1, dans lequel le moyen de mise en correspondance est configuré pour réaliser la mise en correspondance en utilisant un processus de corrélation croisée.

5. Dispositif d'endoscope à ultrasons selon la revendication 1, dans lequel le moyen de mise en correspondance (50) comprend un moyen de calcul de centre de gravité (111) qui est configuré pour calculer le centre de gravité de la première forme de surface et de la deuxième forme de surface.

6. Dispositif d'endoscope à ultrasons selon la revendication 1, dans lequel le moyen de mise en correspondance (50) comprend un moyen de calcul d'axe primaire d'inertie (114) qui est configuré pour calculer l'axe primaire d'inertie de la première forme de surface et de la deuxième forme de surface.

7. Dispositif d'endoscope à ultrasons selon la revendication 1, dans lequel le moyen de mise en correspondance (50) comprend un moyen de calcul de centre de gravité (111) qui est configuré pour calculer le centre de gravité de la première forme de surface et de la deuxième forme de surface, ou un moyen de calcul d'axe primaire d'inertie (114) qui est configuré pour calculer l'axe primaire d'inertie de la première forme de surface et de la deuxième forme de surface, dans lequel le centre de gravité ou l'axe primaire d'inertie est calculé avant le processus de corrélation croisée.

8. Dispositif d'endoscope à ultrasons selon la revendication 1, dans lequel le moyen d'obtention d'images ultrasonores (3) est configuré pour exécuter un balayage de volume de lui-même.

9. Dispositif d'endoscope à ultrasons selon la revendication 8, dans lequel le moyen d'obtention d'images ultrasonores (3) est un vibrateur à ultrasons de type à matrice bidimensionnelle (102) disposé dans une matrice bidimensionnelle avec un vibrateur à ultrasons (22).

# FIG.1

# FIG.2

DIRECTION OF
MINUTE HAND AT
12 O'CLOCK

DIRECTION OF
NORMAL

ROTATE

EP 1 632 184 B1

# FIG.3

EP 1 632 184 B1

# FIG.4

57 : SURFACE SHAPE MEMORY

50

FROM SURFACE
EXTRACTING CIRCUIT 46

59

MUTUAL-
CORRELATION
CIRCUIT

TO COORDINATE
TRANSFORMATION
CIRCUIT 51

FROM SURFACE-SHAPE
ESTIMATING CIRCUIT 48

58 : SURFACE SHAPE MEMORY

EP 1 632 184 B1

# FIG.5

# FIG.6

# FIG.7

63

RE-CUT

65

# FIG.8

# FIG.9

OPTICAL
FIELD-OF-VIEW

EP 1 632 184 B1

# FIG.10

58: SURFACE SHAPE MEMORY

72

57: SURFACE SHAPE MEMORY

71

ROTATE, TRANSLATE, OR ENLARGE/ REDUCE

MATCH

# FIG.11

# FIG.12

RADIAL
SCANNING
SURFACE

ULTRASONIC
BEAMS

81

84

82

RADIAL SCAN

# FIG.14

RADIAL
SCANNING
SURFACE

ULTRASONIC
BEAMS

101

84

102

VOLUME SCAN

# FIG.13

TWIST DIRECTION OF ULTRASONIC ENDOSCOPE

91

93

CONVEX SCAN

92

84

ULTRASONIC BEAMS

CUT POSITION

CONVEX-SCANNING SURFACE

TWIST SCAN

ULTRASONIC TOMOGRAPHIC IMAGE

1  2  3  4  5

DIRECTION FOR TWISTING ULTRASONIC ENDOSCOPE BY HAND

# FIG.15

FROM SURFACE
EXTRACTING CIRCUIT 46

FROM SURFACE-SHAPE
ESTIMATING CIRCUIT 48

*57*

*58*

*110*

*113* TRANSLATION CIRCUIT

*116* ROTATING CIRCUIT

*111* GRAVITY CENTER CALCULATING CIRCUIT

*114* PRIMARY-AXIS-OF-INERTIA CALCULATING CIRCUIT

*117* CROSS-CORRELATION CIRCUIT

*112* GRAVITY CENTER COMPARING CIRCUIT

*115* PRIMARY-AXIS-OF-INERTIA COMPARING CIRCUIT

ROTATE ($\psi$, $\theta$, $\phi$)

TRANSLATE ($\delta X$, $\delta Y$, $\delta Z$)

ENLARGE/
REDUCE $\alpha$

TO COORDINATE
TRANSFORMATION CIRCUIT 51

EP 1 632 184 B1

# FIG.16

FROM SURFACE
EXTRACTING CIRCUIT 46

*57*

FROM SURFACE-SHAPE
ESTIMATING CIRCUIT 48

*58*

*120*

**TRANSLATION CIRCUIT**

*111*

**ROTATING CIRCUIT** *116*

**GRAVITY CENTER CALCULATING CIRCUIT**

*114*

**PRIMARY-AXIS-OF-INERTIA CALCULATING CIRCUIT**

**CROSS-CORRELATION CIRCUIT** *117*

*112*

**GRAVITY CENTER COMPARING CIRCUIT**

*115*

**PRIMARY-AXIS-OF-INERTIA COMPARING CIRCUIT**

ROUGH, ADJUST
TRANSLATE
(δX, δY, δZ)

ROUGH,
ADJUST
ROTATE
(ψ, θ, φ)

**ADJUSTING CIRCUIT** *121*

TO COORDINATE
TRANSFORMATION CIRCUIT 51

EP 1 632 184 B1

# FIG.17

EP 1 632 184 B1

# FIG.18

FIG.19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002017729 A
- JP 2000116655 A
- JP 10000192 A
- JP 11295618 A